# EUROPEAN PATENT APPLICATION

(11) **EP 3 569 687 A1**
(43) Date of publication of application: **20.11.2019**
(21) Application number: 18739210.5
(22) Date of filing: 12.01.2018
(51) Int. Cl.: C12M 1/12, C12M 1/00, C12N 7/00, C12N 13/00, A61L 2/10, B01L 3/00, A61L 2/16

(54) **VIRUS INACTIVATION KIT AND VIRUS INACTIVATION DEVICE**

(30) Priority: 13.01.2017 KR 20170006309; 13.01.2017 KR 20170006300; 13.01.2017 KR 20170006211
(71) Applicant: Nosquest Co., Ltd., Seongnam-Si, Gyeonggi-do 13494 (KR)
(72) Inventor: BOSE, Shambhunath, Yongin-si Gyeonggi-do 16868 (KR); JO, Eung Joon, Yongin-si Gyeonggi-do 16868 (KR)
(74) Representative: Gill Jennings & Every LLP
(86) International application number: PCT/KR2018/000624
(87) International publication number: WO 2018/131936

(57) **Abstract**

According to one embodiment, a virus inactivation kit can comprise: a tube for accommodating a sample containing viruses; a chamber comprising an inner space for accommodating the tube; and at least one light emitting module arranged on one side of the chamber in order to emit light at the tube, wherein the tube can be attached to or detached from the chamber.

## Description

### Technical Field

One or more example embodiments relate to a virus inactivation kit and a virus inactivation device.

### Background Art

Viruses are very threatening to human and animal life. Thus, it is very important to detect viral pathogens at the early stage and analyze characteristics of the viral pathogens. In addition, detection of viruses is important not only in determining treatment strategies, but also in identifying the prevalence of other viruses, such as serotypes or isolates.

For the diagnosis of viral diseases, various testing methods have been carried out, starting with traditional microscopy and up to the recent MALDI-TOF mass spectrometry. In particular, the MALDI-TOF mass spectrometry is a method of measuring a molecular weight of a sample, which is more reliable than typical methods and may analyze characteristics of various viruses. The MALDI-TOF mass spectrometry has advantages of simple and rapid sample preparation, while typical methods require a long time to incubate viruses.

Such method involves sampling of viruses and carrying the viruses to a laboratory. In this process, inactivation of the virus is required because there is a potential risk that the viral pathogen may be exposed to the outside.

As a general inactivation method, pasteurization, dry heat, acidic substances and surfactants have been used. These methods are not suitable for the MALDI-TOF mass spectrometry, which measures the molecular weight of, for example, proteins and DNA fragments, because they can inactivate the virus to prevent replication, but can destroy the virus's proteins.

For the MALDI-TOF mass spectrometry, for example, a method of inactivating by adding UV radiation and chemicals, in which the nucleic acid is inactivated without destroying the protein of the virus, may be preferable.

Accordingly, there is a desire for a kit to inactivating a virus sample using ultraviolet radiation and chemical treatment and simultaneously delivering the virus sample in a process of extracting the virus sample and transporting the virus sample to a laboratory.

The background art described above has been possessed or acquired by the inventor(s) in the course of conceiving the present invention and is not necessarily an art publicly known before filing the present application.

### Disclosure of Invention

### Technical Goals

An aspect provides a virus inactivation kit and a virus inactivation device.

### Technical solutions

According to an aspect, there is provided a virus inactivation kit including a tube configured to accommodate a sample containing viruses, a chamber having an inner space to accommodate the tube, and a light emitting module including at least one light emitting element to emit light to the tube and disposed on one side of the chamber. The tube is detachably attached to the chamber.

The light emitting module may be disposed on an inner circumferential surface of the inner space.

The tube may be formed of at least one material among polytetrafluoroethylene, glass, and quartz.

The light emitting module may be disposed on a perimeter of an outer circumferential surface of the chamber, and the chamber may be formed of at least one material among polytetrafluoroethylene, glass, and quartz.

The chamber may include an entrance portion configured to receive the tube at an upper end, a body portion configured to downwardly extend from the entrance portion and circumferentially cover the inner space, and a lower end portion extending from the body portion to a lower end of the chamber. A vertical height of the chamber may be greater than a width of the chamber. An outer width of the entrance portion may be greater than widths of the body portion and the lower end portion.

The lower end portion may include a battery accommodating recess for accommodating the chamber battery and a battery cover to open and close the battery accommodating recess.

The virus inactivation kit may further include a chamber terminal disposed on an outer wall of the chamber to provide power to the light emitting module.

The chamber may be disposed below the chamber.

The virus inactivation kit may further include a chamber battery accommodated in the chamber, and the chamber battery may be chargeable through the chamber terminal.

The light emitting module may include at least two types of light sources among an ultraviolet (UV)-A light source, a UV-C light source, and a visible light source. The virus inactivation kit may further include a light emitting controller configured to simultaneously or selectively operate the at least two types of light sources and an interface installed to be exposed to an outside of the virus inactivation kit to receive a user command transmitted to the light emitting controller.

According to another aspect, there is also provided a virus inactivation apparatus including a tube configured to accommodate a sample containing viruses, a chamber having an inner space to accommodate the tube, at least one light emitting module disposed on one side of the chamber to emit light to the tube, a chamber battery accommodated in the chamber, a chamber terminal disposed on an outer wall of the chamber to provide power to the light emitting module, a case including an accommodating port configured to accommodate the chamber, and a case terminal disposed in the accommodating port and configured to contact the chamber terminal to provide power to the chamber. wherein the case terminal is configured to provide the power to the chamber battery and the light emitting module.

The accommodating port may be recessed from a top surface of the case, and the case terminal may be disposed on a bottom surface of the accommodating port.

The virus inactivation apparatus may further include a power source line configured to receive power from an outside and provide the power to the case terminal.

The virus inactivation apparatus may further include a case battery configured to provide power to the case terminal.

The virus inactivation apparatus may further include a controller configured to sense a charge amount of the chamber battery or the case battery and a display configured to display the change amount sensed by the controller.

The virus inactivation apparatus may further include a second accommodating port configured to accommodate the tube.

The virus inactivation apparatus may further include at least one light emitting module disposed on an inner wall of the second accommodating port or inside the case to emit light toward an inner space of the second accommodating port.

According to another aspect, there is also provided a virus inactivation apparatus for inactivating viruses contained in a sample, the apparatus including a virus inactivation kit configured to accommodate a sample containing viruses and a light emitting module including a plurality of light emitting elements to emit light to the virus inactivation kit, wherein the plurality of light emitting elements includes at least two types of light sources among a UV-A light source that emits a UV-A ray, a UV-C light source that emits a UV-C ray, and a visible light source that emits a visible ray, wherein the virus inactivation apparatus further includes a light emitting controller configured to simultaneously or selectively operate the at least two types of light sources and an interface installed to be exposed to an outside of the virus inactivation kit to receive a user command transmitted to the light emitting controller, and wherein the light emitting controller is configured to selectively control a type of a ray emitted by the light emitting module based on a type of chemical added to the sample.

A space between light emitting elements emitting a same type of ray among the plurality of light emitting elements may be greater than a space between light emitting elements emitting different types of rays.

The light emitting module may further include a light emitting panel having a length greater than a width, and a first light source that emits one type of ray and a second light source that emits another type of ray among the plurality of light emitting elements may be alternately arranged in a longitudinal direction of the light emitting panel.

The plurality of light emitting elements may be arranged at preset intervals based on a grid pattern.

The plurality of light emitting elements may be arranged at preset intervals, and a row or column of the arrangement may be in a zigzag pattern.

The light emitting module further may include a light emitting panel having a cylindrical shape.

The plurality of light emitting elements may be arranged on an inner circumferential surface of the light emitting panel at preset intervals.

The plurality of light emitting elements may include at least one type of light source between the UV-C light source and the visible light source, and the tube may include at least one chemical selected from methylene blue, thiazole orange, thioperielium, and dipyramol.

The plurality of light emitting elements may include at least one type of light source between the UV-A light source and the UV-C light source, and the tube may include at least one chemical selected from 4-aminomethylenetrioxalene and riboflavin. Brief Description of Drawings
FIG. 1 is a diagram illustrating a virus inactivation device according to an example embodiment.
FIG. 2A is a perspective view illustrating a virus inactivation kit according to an example embodiment.
FIG. 2B is a cross-sectional view illustrating a virus inactivation kit according to an example embodiment.
FIG. 3A is a perspective view illustrating a virus inactivation kit according to an example embodiment.
FIG. 3B is a cross-sectional view illustrating a virus inactivation kit according to an example embodiment.
FIG. 4 is a front view illustrating a light emitting module according to an example embodiment.
FIG. 5 is a front view illustrating a light emitting module according to an example embodiment.
FIG. 6 is a perspective view illustrating a light emitting module according to an example embodiment.
FIG. 7 is a cross-sectional view illustrating a virus inactivation kit according to an example embodiment.
FIG. 8 is a cross-sectional view illustrating a virus inactivation kit according to an example embodiment.
FIG. 9 is a diagram illustrating a virus inactivation device according to an example embodiment.
FIG. 10 is a diagram illustrating a virus inactivation device according to an example embodiment.

### Best Mode for Carrying Out the Invention

Hereinafter, example embodiments will be described in detail with reference to the accompanying drawings. It should be understood, however, that there is no intent to limit this disclosure to the particular example embodiments disclosed. On the contrary, example embodiments are to cover all modifications, equivalents, and alternatives falling within the scope of the example embodiments.

Although terms such as "first," "second," and "third" may be used herein to describe various members, components, regions, layers, or sections, these members, components, regions, layers, or sections are not to be limited by these terms. Throughout the specification, when an element, such as a layer, region, or substrate, is described as being "on," "connected to," or "coupled to" another element, it may be directly "on," "connected to," or "coupled to" the other element, or there may be one or more other elements intervening therebetween.

Regarding the reference numerals assigned to the elements in the drawings, it should be noted that the same elements will be designated by the same reference numerals, wherever possible, even though they are shown in different drawings. Also, in the description of embodiments, detailed description of well-known related structures or functions will be omitted when it is deemed that such description will cause ambiguous interpretation of the present disclosure.

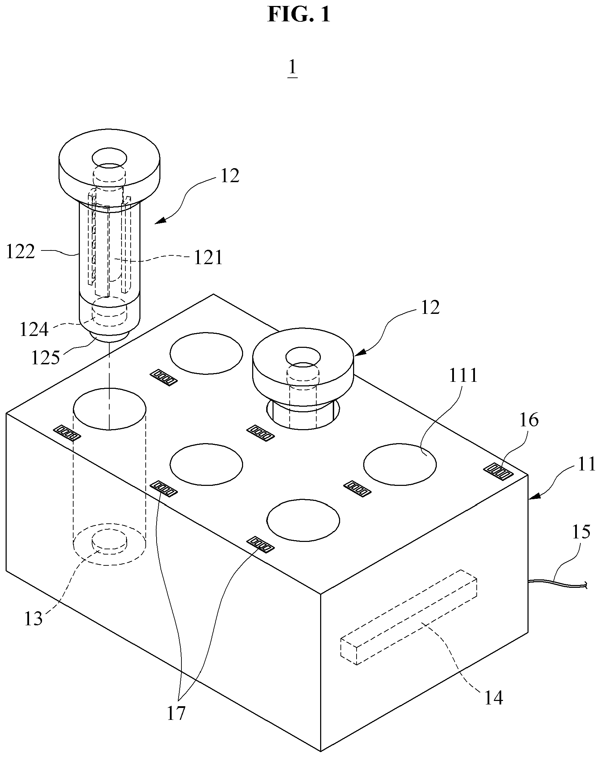

FIG. 1 is a diagram illustrating a virus inactivation device 1 according to an example embodiment.

Referring to FIG. 1, the virus inactivation device 1 may be a device for collecting a sample containing viruses, adding chemicals selectively to the sample, and then storing, transporting, and inactivating the sample. The virus inactivation device 1 may include a case 11, a virus inactivation kit 12, a case terminal 13, a case battery 14, a power source line 15, a case controller (not shown), and a display 16, 17.

The case 11 may supply power to the virus inactivation kit 12 or charge a chamber battery 124 of the virus inactivation kit 12. For example, the case 11 may be portable and may also be fixedly installed in a transportation such as a vehicle including the virus inactivation device 1.

As shown in FIG. 1, the case 11 may be formed as a rectangular parallelepiped, but the case 11 may have various shapes of appearance.

The case 11 may include an accommodating port 111 to arrange the virus inactivation kit 12 in the case 11.

The accommodating port 111 may be a space for accommodating the virus inactivation kit 12 and recessed from a top surface of the case 11. The accommodating port 111 may be formed in a shape corresponding to an outer shape of the virus inactivation kit 12. For example, the accommodating port 111 may be an inner space in a cylindrical shape as shown in FIG. 1.

Also, the accommodating port 111 may be provided as a plurality of accommodating ports 111. Although FIG. 1 illustrates six accommodating ports 111, the accommodating port 111 may also be provided as a single accommodating port 111.

The virus inactivation kit 12 may be a kit for inactivating a sample including a virus sample. Also, the virus inactivation kit 12 may be a device for inactivating viruses of the sample, and then storing and transporting the sample. For example, chemicals used for virus inactivation may be added to the sample.

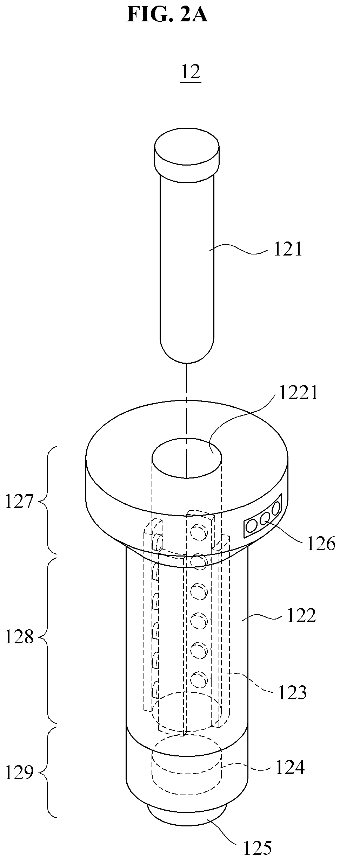

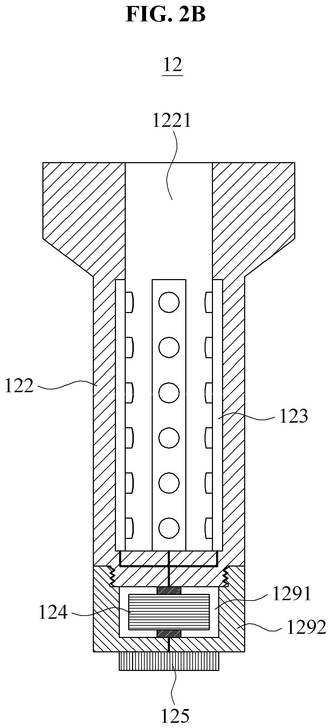

The virus inactivation kit 12 may include a tube 121, a chamber 122, a light emitting module 123, the chamber battery 124, a chamber terminal 125 and an interface 126 (refer to FIG. 2A). Related description will be made with reference to FIG. 2.

The case terminal 13 may be a terminal for supplying power to the virus inactivation kit 12 and charging the chamber battery 124 of the virus inactivation kit 12 and disposed in the accommodating port 111. For example, the case terminal 13 may be disposed on a bottom surface of the accommodating port 111 as shown in FIG. 1.

The case terminal 13 may contact the chamber terminal 125 of the virus inactivation kit 12 to provide the power. Depending on an example, the case 11 may include a charger for wireless charging the chamber battery 124 without being in direct contact with the chamber terminal 125.

The case battery 14 may be a battery disposed in the case 11, The case battery 14 may be installed external or internal to the case 11 and provide power supplied to the virus inactivation kit 12. By using the case battery 14, the power may be supplied to the virus inactivation kit 12 while the case 11 is being carried externally.

The power source line 15 may supply power for operating the virus inactivation device 1 from an external power source to the case 11. Through this, the case battery 14 may be charged.

The case controller (not shown) may control the power transmitted from the case terminal 13 to the virus inactivation kit 12 and sense charge amounts of the case battery 14 and the chamber battery 124.

The display 16, 17 may be a device for displaying the charge amounts of the case battery 14 and the chamber battery 124 sensed by the case controller (not shown) and disposed on an outer surface of the case 11.

As shown in FIG. 1, the display 16, 17 may include a case battery lamp 16 that indicates the charge amount of the case battery 14 and a chamber battery lamp 17 that indicates the charge amount of the chamber battery 124 of the virus inactivation kit 12 in the accommodating port 111.

Referring to FIG. 1, the case battery lamp 16 may be disposed at a right upper end of the top surface of the case 11. Also, the chamber battery lamp 17 may be disposed near an entrance of the accommodating port 111 on the top surface of the case 11 for each of the accommodating ports 111.

FIGS. 2A and 2B are diagrams illustrating a virus inactivation kit 12 according to an example embodiment. Specifically, FIG. 2A is a perspective view illustrating a virus inactivation kit according to an example embodiment and FIG. 2B is a cross-sectional view illustrating a virus inactivation kit according to an example embodiment.

Referring to FIGS. 2A and 2B, the virus inactivation kit 12 may be a device for inactivating a sample containing a virus sample. The virus inactivation kit 12 may be grasped and carried by a user. The virus inactivation kit 12 may be accommodated in the accommodating port 111 of the case 11 and operate using applied power. For example, the virus inactivation kit 12 may be separated from the case 11 and operate independently.

The virus inactivation kit 12 may include the tube 121, the chamber 122, the light emitting module 123, the chamber battery 124, the chamber terminal 125, a light emission controller (not shown) and the interface 126.

The tube 121 may be a container for storing a sample containing viruses and detachably attached to an inner space 1221 of the chamber 122. Here, the sample may be a living tissue or blood collected from an animal infected with a virus, and a chemical may optionally be added to the sample. Chemicals will be described with reference to Table 1 to be described later.

For example, the tube 121 may be disposable. Also, a cover may be attached from an upper end so that a gas tight is maintained after the sample is stored. The cover may have a material and/or a color that blocks light except light emitted from the light emitting module 123.

Also, the tube 121 may be made of a material having a relatively high light-transmittance so as to pass the light from the light emitting module 123 to inactivate the virus of the sample. The tube 121 may be formed of at least one material among, for example, polytetrafluoroethylene, glass, and quartz.

The chamber 122 may accommodate the tube 121 and inactivate the viruses of the sample in the tube 121. As shown in FIGS. 2A and 2B, the chamber 122 may have a vertical height greater than a width and may be in a columnar shape of which a cross section is circular. The chamber 122 may be formed in various width and height ratios, and the cross section may be in various shapes such as a triangle and a square.

The chamber 122 may have the inner space 1221 for accommodating the sample. Referring to FIG. 2A, the chamber 122 may include an entrance portion 127 that receives the tube 121 at an upper end, a body portion 128 that extends from the entrance portion 127 to the inner space 1221, and a lower end portion 129 that extends from the body portion 128 to a lower end of the chamber 122.

The inner space 1221 may be a space recessed from a center of the top surface of the chamber 122 and extend from the entrance portion 127 to the lower end of the body portion 128. As shown in FIGS. 2A and 2B, the inner space 1221 may be in a cylindrical shape and have a diameter or a width greater than that of the tube 121.

An outer width of the entrance portion 127 of the chamber 122 may be greater than outer widths of the body portion 128 and the lower end portion 129. For example, as shown in FIG. 2A, a diameter of an outer circumference of the entrance portion 127 may protrude and be formed to be greater than the body portion 128 and the lower end portion 129, so that the outer surface of a section connecting the body portion 128 and the entrance portion 127 is inclined.

As such, with the chamber 122 having the greater outer width of the entrance portion 127, the user may easily grasp the chamber 122 without missing. When the virus inactivation kit 12 is disposed in the accommodating port 111 of the case 11 as shown in FIG. 1, the user may grasp a protruding portion of the entrance portion 127, thereby easily separating the virus inactivation kit 12 from the case 11.

The light emitting module 123 may be a device for emitting light toward the tube 121 and include a panel and a plurality of light emitting elements arranged on the panel.

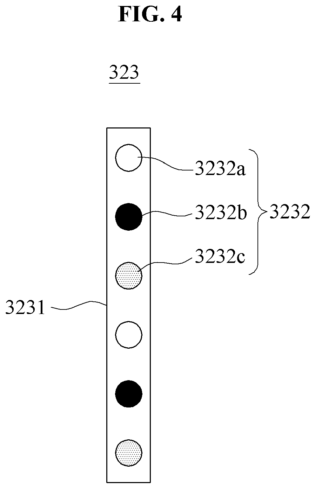

The light emitting module 123 may include at least one of an ultraviolet (UV)-A light source, a UV-C light source, and a visible light source. A configuration of the light emitting module 123 will be further described with reference to FIGS. 4 through 6.

For example, as shown in FIGS. 2A and 2B, the light emitting module 123 may include the plurality of light emitting elements arranged in a line on an outer surface of the panel. Also, at least one light emitting module 123 may be disposed on an outer circumferential surface of the inner space 1221 to face a center of the inner space 1221.

As shown in FIGS. 2A and 2B, a plurality of light emitting modules 123 may be arranged along an inner circumference of the inner space 1221 at preset intervals. It should be understood, however, that a location, shape, and number of light emitting modules 123 installed in the virus inactivation kit 12 can be arbitrarily selected by a person skilled in the art while performing the installation. Examples of the light emitting module 123 will be made with reference to FIGS. 4 through 8.

The chamber battery 124 may supply power for operating the light emitting module 123. As shown in FIG. 2B, the chamber battery 124 may be accommodated in a battery accommodating recess 1291 formed in the lower end portion 129.

The chamber battery 124 may be replaceable. To replace the chamber battery 124, the battery accommodating recess 1291 may be separated from the lower end portion 129. Also, a battery cover 1292 may be provided to open and close the battery accommodating recess 1291.

The chamber terminal 125 may be a terminal to receive the power from the case terminal 13 of the case 11 and disposed on an outer wall of the chamber 122. For example, as shown in FIGS. 2A and 2B, the chamber terminal 125 may be disposed on a lower surface of the lower end portion 129.

In this case, the case terminal 13 may be disposed on a lower surface of the accommodating port 111. The virus inactivation kit 12 may be located in the accommodating port 111, so that the chamber terminal 125 naturally contacts the case terminal 13. Through this, the virus inactivation kit 12 may receive the power, operate the light emitting module 123, and charge the chamber battery 124.

The light emission controller (not shown) may control an operation of the light emitting module 123. When the light emitting module 123 includes at least two types of light sources, the light emission controller (not shown) may simultaneously or selectively control the at least two types of light sources.

The interface 126 may be a device for receiving a command from a user and transmitting the command to the light emission controller (not shown). The interface 126 may be exposed to an outside of the chamber 122 so as to be operated by the user. As shown in FIG. 2A, the interface 126 may be installed on the outer surface of the entrance portion 127.

In the above structure, the user may manipulate the interface 126 installed outside the chamber 122 to output an input signal that controls a desired type of light source and a power source of the light emitting module 123. The interface 125 may include a plurality of buttons corresponding to types of light sources or a plurality of buttons corresponding to chemicals to be added to the sample.

The input signal may be transmitted to the light emission controller (not shown). Based on the input signal, the light emission controller (not shown) may operate the light emitting module 123. For example, the light emission controller (not shown) may selectively control a type of light emitted from the light emitting module 123.

In the tube 121, a chemical may be selectively added to inactivate viruses. In this case, a type of light emitted from the light emitting module 123 may be selectively controlled to effectively inactivate the virus based on the added chemical.

Table 1 represents chemicals to be added to the sample and light sources corresponding to the chemicals.

**[Table 1]**

| **No.** | **Chemical** | **UV A** | **UVC** | **Visible light** | **No light** |
|---|---|---|---|---|---|
| 1 | Methylene blue | | **Y** | **Y** | |
| 2 | 4'-Aminomethyltrioxsalen | **Y** | **Y** | | |
| 3 | Riboflavin | **Y** | **Y** | | |
| 4 | Thiazole Orange | | **Y** | **Y** | |
| 5 | Thiopyrylium | | **Y** | **Y** | |
| 6 | Dipyridamole | | **Y** | **Y** | |

As shown in Table 1, one or more chemicals of methylene blue, 4'-aminomethyltrioxsalen, riboflavin, thiazole Orange, thiopyrylium, and dipyridamole may be added to the sample. A type of light emitting element to be controlled based on the corresponding chemical may be confirmed in Table 1. For example, according to Table 1, it can be configured that when methylene blue is added, a UV-C light source and/or a visible light source may be used to inactivate the virus of the sample.

The UV-A light source and/or the visible light source may activate the corresponding chemical of Table 1. The activated chemical may react with the virus to inactivate the virus. After the activated chemical inactivates the viruses, the UV-A light source and/or the visible light source may not emit the UV-A ray and/or the visible ray. As such, once the virus is inactivated, the UV-C light source may emit the UV-C ray to maintain the inactivated state of the virus.

As an example, the plurality of light emitting elements may include at least one type of light source between the UV-C light source and the visible light source, and the virus inactivation kit may include at least one chemical among methylene blue, thiazole orange, thioperielium, and dipyramol. As another example, the plurality of light emitting elements may include at least one type of light source between the UV-A light source and the UV-C light source and the virus inactivation kit may include at least one chemical between 4-aminomethylenetrioxalene and riboflavin.

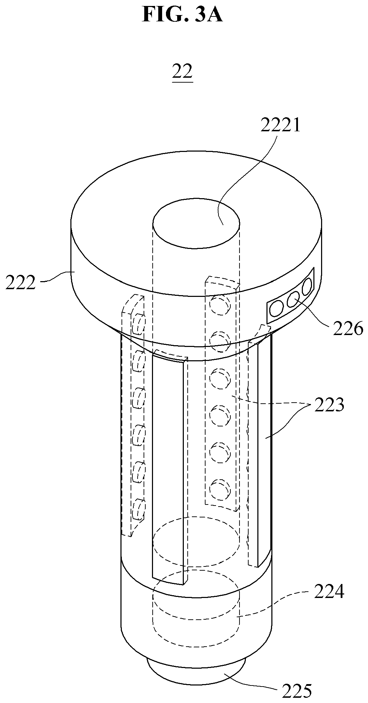

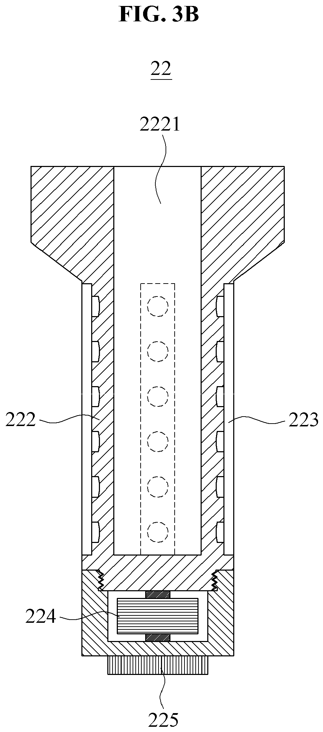

FIGS. 3A and 3B are diagrams illustrating a virus inactivation kit 22 according to an example embodiment. Specifically, FIG. 3A is a perspective view illustrating a virus inactivation kit according to an example embodiment and FIG. 3B is a cross-sectional view illustrating a virus inactivation kit according to an example embodiment.

Referring to FIGS. 3A and 3B, the virus inactivation kit 22 may include a tube, a chamber 222 including an inner space 2221, a light emitting module 223, a chamber battery 224, a chamber terminal 225, and an interface 226.

The light emitting module 223 may be attached to an outer surface of the chamber 222 instead of the inner space 2221. For example, as shown in FIGS. 3A and 3B, a plurality of light emitting modules 223 may be arranged along an outer circumference of a body portion of the chamber 222 at preset intervals.

The light emitting module 223 may also be disposed between the outer surface of the chamber 222 and the inner space 2221 in addition to the outer surface of the chamber 222.

As such, when the light emitting module 223 is disposed external to the inner space 2221 of the chamber 222, the chamber 222 may be formed of a material having a relatively high light-transmittance such that the light is emitted from the light emitting module 223 toward a sample of the inner space 2221. For example, the chamber 222 may be formed of at least one material among polytetrafluoroethylene, glass, and quartz.

FIG. 4 is a front view illustrating a light emitting module according to an example embodiment.

Referring to FIG. 4, a light emitting module 323 may include a panel 3231 and a plurality of light emitting elements 3232. Here, the panel 3231 may be a thin substrate on which the plurality of light emitting elements 3232 is installed.

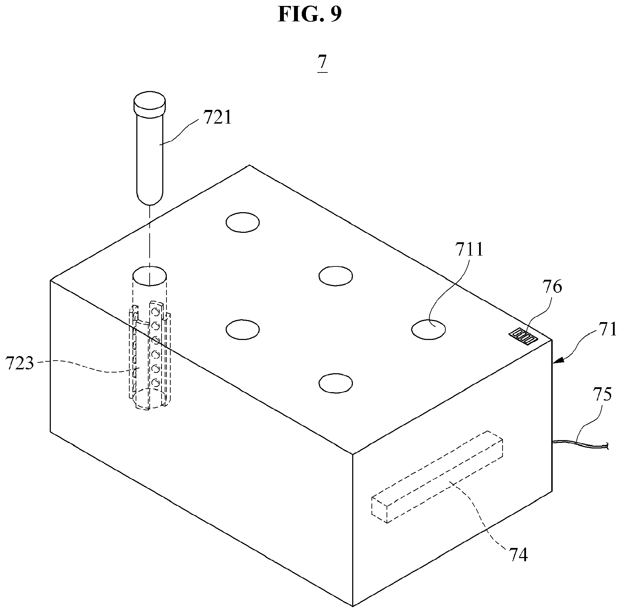

For example, the light emitting module 323 may be installed in the virus inactivation kit 12 as described with reference to FIGS. 1 through 3B and also be installed in the accommodating port 111 of the case 11 as described with reference to FIG. 9.

The plurality of light emitting elements 3232 may be elements that emit light and are installed on an outer surface of the panel 3231, and may be light emitting diodes. The plurality of light emitting elements 3232 may include at least one type of light source among a UV-A light source 3232a, a UV-C light source 3232b, and a visible light source 3232c.

The UV-A light source 3232a may be an element that emits a UV ray in a range between 315 nanometers (nm) and 400 nm. The UV-C light source 3232b may be an element that emits a UV ray in a domain ranging between 200 nm and 280 nm. The visible light source 3232c may be an element that emits a visible ray in a range between 400 nm and 700 nm.

For example, the plurality of light emitting elements 3232 may include one type of light source. Also, as shown in FIG. 4, the plurality of light emitting elements 3232 may include the UV-A light source 3232a, the UV-C light source 3232b, and the visible light source 3232b.

As shown in FIG. 4, the panel 3231 may be elongated in a longitudinal direction, so that the plurality of light emitting elements 3232 is arranged in a line on the panel 3231. When the light emitting module 323 includes at least two types of light sources, the plurality of light emitting elements may be alternately arranged in the longitudinal direction.

As such, the plurality of light emitting elements 3232 may be alternately arranged. In this example, when the light emitting module 323 emits light toward the tube 121, a specific light source may be uniformly radiated to the entire sample without concentrating on one part.

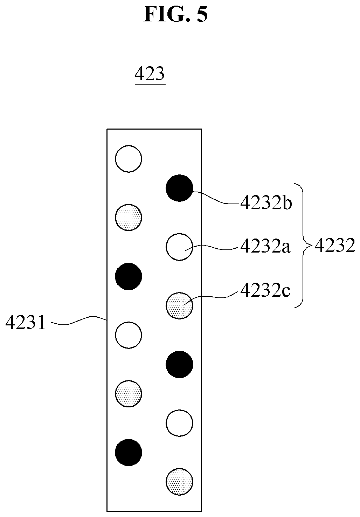

FIG. 5 is a front view illustrating a light emitting module according to an example embodiment.

Referring to FIG. 5, a light emitting module 423 may include a panel 4231 and a plurality of light emitting elements 4232. Here, the plurality of light emitting elements 4232 may be lengthily arranged in two rows in a longitudinal direction to be installed on the panel 4231.

When the plurality of light emitting elements 4232 is arranged in at least two rows, the plurality of light emitting elements 4232 may be arranged at preset intervals based on a grid pattern. Also, as shown in FIG. 5, the plurality of light emitting elements 4232 may be arranged in a zigzag pattern in which one row or column is reciprocated.

Also, as shown in FIG. 5, when the plurality of light emitting elements includes a UV-A light source 4232a, a UV-C light 4232b and a visible light source 4232c, an interval between a same type of neighboring light sources may be greater than an interval between different types of neighboring light sources.

Accordingly, when the light emitting module 423 including at least two types of light sources emits light toward a sample, a specific light source may be uniformly irradiated onto the entire sample without being concentrating to one part.

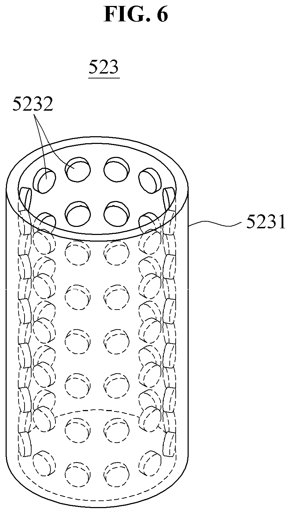

FIG. 6 is a perspective view illustrating a light emitting module according to an example embodiment.

Referring to FIG. 6, a light emitting module 523 may include a light emitting panel 5231 and a plurality of light emitting elements 5232.

As shown in FIG. 6, the light emitting panel 5231 may have a cylindrical shape and include a thin substrate. Also, the plurality of light emitting elements 5232 may be arranged on an inner circumferential surface of the light emitting panel 5231 at preset intervals.

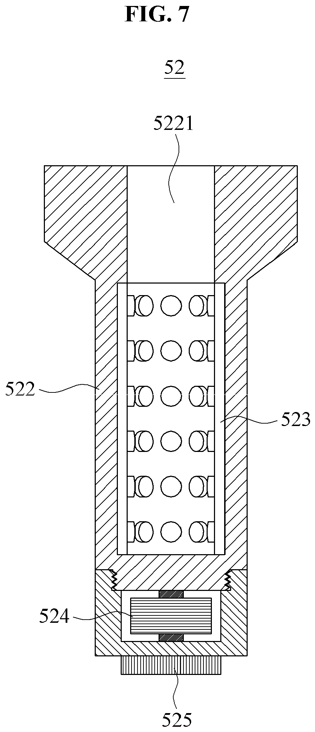

For example, the light emitting module 523 may be installed in the virus inactivation kit 12 as shown in FIG. 1. Also, the light emitting module 523 may be installed in the accommodating port 111 of the case 11. Related description will be made with reference to FIGS. 7, 8, and 10.

In the above structure, the tube 121 containing a sample may be located at a center of an inside of the light emitting module 523, so that light may be uniformly irradiated onto the entire sample.

FIG. 7 is a cross-sectional view illustrating a virus inactivation kit according to an example embodiment.

Referring to FIG. 7, a virus inactivation kit 52 may include a tube, a chamber 522 including an inner space 5221, a light emitting module 523, a chamber battery 524, and a chamber terminal 525.

The light emitting module 523 may be installed on an inner circumferential surface of the inner space 5221 of the chamber 522. The plurality of light emitting elements 5232 of the light emitting module 523 may be densely arranged along a perimeter of an inner circumferential surface of the inner space 5221 to emit light toward a center of the inner space 522. Through this, a sample inserted in the inner space 522 may be effectively inactivated.

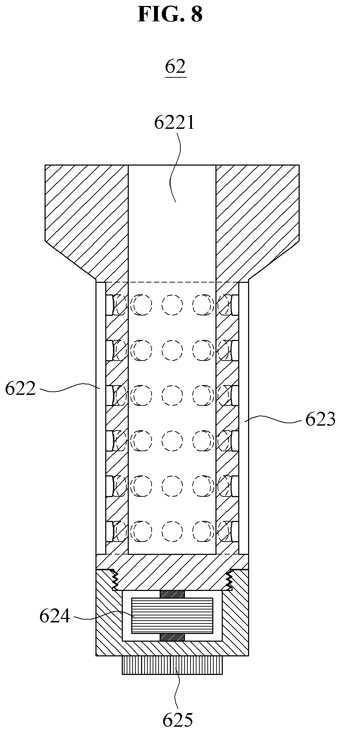

FIG. 8 is a cross-sectional view illustrating a virus inactivation kit according to an example embodiment.

Referring to FIG. 8, a virus inactivation kit 62 may include a tube, a chamber 622 including an inner space 6221, a light emitting module 623, a chamber battery 624, and a chamber terminal 625.

The light emitting module 623 may be installed on an outer wall of the chamber 622. The light emitting module 623 may be installed along a circumference of the outer wall of the chamber 522, so that a plurality of light emitting elements are densely arranged on the circumference of the outer wall of the chamber 522.

FIG. 9 is a diagram illustrating a virus inactivation device according to an example embodiment.

Referring to FIG. 9, a virus inactivation device 7 may be a device for inactivating viruses of a sample by accepting a virus inactivation kit 721. Unlike the virus inactivation device 1 accepting the virus inactivation kit 12 including the chamber 12 and the tube 121 in the example of FIG. 1, the virus inactivation device may accept the virus inactivation kit including a tube 721.

The virus inactivation device 7 may include a case 71 including an accommodating port 711, the tube 721, a light emitting module 723, a case battery 74, a power source line 75, a case controller (not shown), and a display 76.

The accommodating port 711 may accommodate the tube 721 and may be, for example, a space formed in a cylindrical shape. The tube 721 may be a space in which a sample containing viruses is stored. The tube 121 of FIG. 2A may be used as the tube 721.

The light emitting module 723 may be installed in the accommodating port 711 and emit light toward the tube 721 inserted in the accommodating port 711. For example, as shown in FIG. 9, a plurality of light emitting modules 723 may be arranged in a line and installed on an inner wall of the accommodating port 711.

The light emitting module 723 may be installed in the case 71, that is, an outside of an inner circumferential surface of the accommodating port 711 to emit the light into the accommodating port 711. In this case, the tube 71 may be made of a material having a relatively high light-transmittance. For example, the tube 71 may be formed of at least one material among polytetrafluoroethylene, glass, and quartz.

The case battery 74 may be installed in the case 71 to provide power for operating the light emitting module 723.

The power source line 75 may supply power for operating the light emitting module 723 from an external power source to the case 71. Through this, the case battery 74 may be charged.

The case controller (not shown) may sense a change amount of the case battery 74 and controls an operation of the light emitting module 723. When the light emitting module 723 includes at least two types of light sources, the light emission controller (not shown) may simultaneously or selectively control the at least two types of light sources.

The display 76 may display the charge amount of the case battery 74sensed by the case controller (not shown).

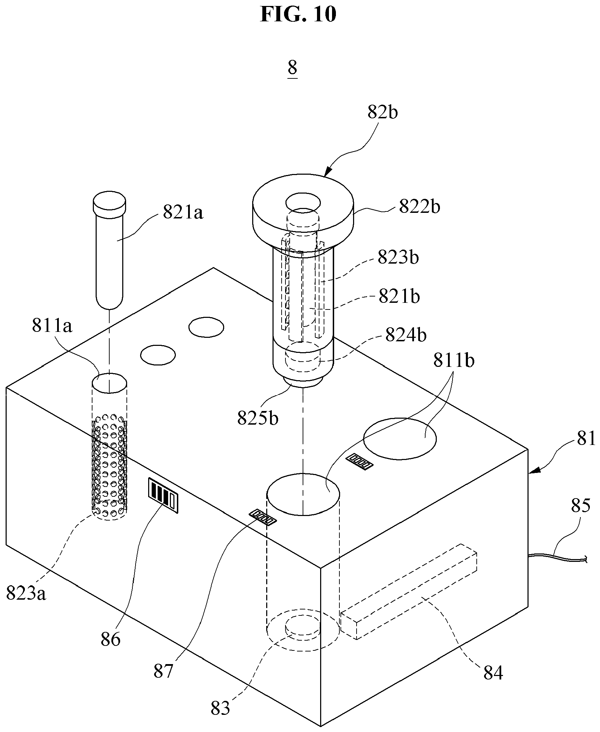

FIG. 10 is a diagram illustrating a virus inactivation device according to an example embodiment.

Referring to FIG. 10, a virus inactivation device 8 may be a device for accommodating a virus inactivation kit 82b and inactivating viruses of a sample, and may accommodate a tube 821a alone as well as the virus inactivation kit 82b.

The virus inactivation device 8 may include a case 81, the tube 821a, the virus inactivation kit 82b, a case terminal 83, a light emitting module 823a, a case battery 84, a power source line 85, a controller (not shown), and a display 86, 87.

The case 81 may accommodate the virus inactivation kit 82b, supply power to the virus inactivation kit 82b, and charge a chamber battery 824b of the virus inactivation kit 82b.

The case 81 may directly the tube 821a containing the sample including the viruses, and may also include a plurality of accommodating ports 811a and 811b to accommodate the virus inactivation kit 82b and the tube 821a.

The accommodating port 811a, 811b may be a space recessed from a top surface of the case 81. The accommodating ports 811a and 811b may include a first accommodating port 811a to accommodate the tube 821a and a second accommodating port 811b to accommodate the virus inactivation kit 82b.

The first accommodating port 811a may be a port for accommodating the tube 821a and be a space in a cylindrical shape as shown in FIG. 10. The light emitting module 823a may be installed on an inner wall of first accommodating port 811a.

The second accommodating port 811b may be a port for accommodating the virus inactivation kit 82b and be a space in a cylindrical shape as shown in FIG. 10. A width of the second accommodating port 811b may be greater than a width of the first accommodating port 811a. For example, the case terminal 83 may be installed in the second accommodating port 811b.

The tube 821a may be a container in which the sample including viruses is stored, be a disposable container, be replaceable, and be the tube 121 of FIG. 2A. For example, a chemical for inactivating the viruses may be added to the sample.

The virus inactivation kit 82b may be a kit for inactivating the viruses of the sample, and then storing and transporting the sample. The virus inactivation kit 82b may be accommodated in the second accommodating port 811b of the case 81, operate by receiving power through the chamber battery 824b, operate independently, and be provided in the structure of the virus inactivation kit 12 illustrated in FIG. 2A.

The virus inactivation kit 82b may include the tube 821a, a chamber 822b, a light emitting module 823b, the chamber battery 824b, a chamber terminal 825b, and an interface.

The configuration of the virus inactivation kit 82b may also be the same as the virus inactivation kit 12 of FIG. 2A. However, it should be understood that the configuration of the light emitting module 823b may be freely modified in its position, arrangement, light source type, and the like, as in the foregoing examples.

The case terminal 83 may be a terminal for supplying power to the virus inactivation kit 82b and charging the chamber battery 824b of the virus inactivation kit 82b. The case terminal 83 may be disposed in the second accommodating port 811b. For example, the case terminal 83 may be disposed on a bottom surface of the second accommodating port 811b to contact the chamber terminal 825b as shown in FIG. 1.

The light emitting module 823a may be a device for emitting light toward the sample and disposed in the first accommodating port 811a. As shown in FIG. 10, the light emitting module 823a having a cylindrical panel may be installed on an inner circumferential surface of the first accommodating port 811a. Also, the light emitting module 823a may have the same configuration as the light emitting module 823b installed in the virus inactivation kit 82b and may be interchangeable.

For example, the light emitting module 823a may be installed inside the first accommodating port 811a and may also be installed outside an inner wall of the first accommodating port 811a, that is, inside the case 81. In this case, the case 81 may be formed of a material having a high light-transmittance.

The light emitting module 823a, 823b may be freely modified in its position, arrangement, light source type, and the like, as in the foregoing examples.

The case battery 84 may be a battery disposed in the case 81. The case battery 84 may provide power supplied to the virus inactivation kit 82b through the case terminal 83 and provide power for operating the light emitting module 823a installed in the first accommodating port 811a. By using the case battery 84, the virus inactivation device 8 may be operated while the case 81 is being carried externally.

The power source line 85 may supply power from an external power source to the case 81 so as to operate the virus inactivation device 8 and charge the case battery 84. Also, the power source line 85 may provide power for operating the light emitting module 823a installed in the first accommodating port 811a.

The controller (not shown) may control the power transmitted from the case terminal 83 installed in the second accommodating port 811b to the virus inactivation device 1, and control an operation of the light emitting module 823 installed in the first accommodating port 811a. When the light emitting module 823a includes at least two types of light sources, the controller (not shown) may simultaneously or selectively control the at least two types of light sources.

Also, the controller (not shown) may sense charge amounts of the chamber battery 824b of the virus inactivation kit 8 and the case battery 84.

The display 86, 87 may be a device for displaying charge amounts of the case battery 84 and the chamber battery sensed by the case controller (not shown) and installed on an outer surface of the case 81.

For example, similar to the example of FIG. 1, the display 86, 87 may include a case battery lamp 86 that indicates the charge amount of the case battery 84 and a chamber battery lamp 87 that indicates the charge amount of the chamber battery 824b of the virus inactivation kit 82b in the accommodating port 811b.

Referring to FIG. 10, the case battery lamp 86 may be disposed on a front surface of the case 81. Also, the chamber battery lamp 87 may be disposed near an entrance of the accommodating port 811b on the top surface of the case 81 for each of the second accommodating ports 811b. However, positions of the case battery lamp 86 and the chamber battery lamp 87 are not limited thereto.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components or their equivalents.

Accordingly, other implementations are within the scope of the following claims.

## Claims

1. A virus inactivation kit comprising:
a tube configured to accommodate a sample containing viruses;
a chamber having an inner space to accommodate the tube; and
a light emitting module comprising at least one light emitting element to emit light to the tube and disposed on one side of the chamber,
wherein the tube is detachably attached to the chamber.

2. The virus inactivation kit of claim 1, wherein the chamber comprises:
an entrance portion configured to receive the tube at an upper end;
a body portion configured to downwardly extend from the entrance portion and circumferentially cover the inner space; and
a lower end portion extending from the body portion to a lower end of the chamber,
a vertical height of the chamber is greater than a width of the chamber, and
an outer width of the entrance portion is greater than widths of the body portion and the lower end portion.

3. The virus inactivation kit of claim 1, further comprising:
a chamber terminal disposed on an outer wall of the chamber to provide power to the light emitting module.

4. The virus inactivation kit of claim 3, wherein the chamber is disposed below the chamber.

5. The virus inactivation kit of claim 3, further comprising:
a chamber battery accommodated in the chamber,
wherein the chamber battery is chargeable through the chamber terminal.

6. A virus inactivation apparatus comprising:
a tube configured to accommodate a sample containing viruses;
a chamber having an inner space to accommodate the tube; and
at least one light emitting module disposed on one side of the chamber to emit light to the tube;
a chamber battery accommodated in the chamber;
a chamber terminal disposed on an outer wall of the chamber to provide power to the light emitting module;
a case including a first accommodating port configured to accommodate the chamber; and
a case terminal disposed in the first accommodating port and configured to contact the chamber terminal to provide power to the chamber,
wherein the case terminal is configured to provide the power to the chamber battery and the light emitting module.

7. The virus inactivation apparatus of claim 6, wherein the first accommodating port is recessed from a top surface of the case and
the case terminal is disposed on a bottom surface of the first accommodating port.

8. The virus inactivation apparatus of claim 6, further comprising:
a power source line configured to receive power from an outside and provide the power to the case terminal.

9. The virus inactivation apparatus of claim 6, further comprising:
a case battery configured to provide power to the case terminal.

10. The virus inactivation apparatus of claim 9, further comprising:
a controller configured to sense a charge amount of the chamber battery or the case battery; and
a display configured to display the change amount sensed by the controller.

11. The virus inactivation apparatus of claim 6, further comprising:
a second accommodating port configured to accommodate the tube.

12. The virus inactivation apparatus of claim 11, further comprising:
at least one light emitting module disposed on an inner wall of the second accommodating port or inside the case to emit light toward an inner space of the second accommodating port.

13. A virus inactivation apparatus for inactivating viruses contained in a sample, the apparatus comprising:
a virus inactivation kit configured to accommodate a sample containing viruses; and
a light emitting module comprising a plurality of light emitting elements to emit light to the virus inactivation kit,
wherein the plurality of light emitting elements comprises at least two types of light sources among an ultraviolet (UV)-A light source that emits a UV-A ray, a UV-C light source that emits a UV-C ray, and a visible light source that emits a visible ray,
wherein the virus inactivation apparatus further comprises:
a light emitting controller configured to simultaneously or selectively operate the at least two types of light sources; and
an interface installed to be exposed to an outside of the virus inactivation kit to receive a user command transmitted to the light emitting controller, and
wherein the light emitting controller is configured to selectively control a type of a ray emitted by the light emitting module based on a type of chemical added to the sample.

14. The virus inactivation apparatus of claim 13, wherein a space between light emitting elements emitting a same type of ray among the plurality of light emitting elements is greater than a space between light emitting elements emitting different types of rays.

15. The virus inactivation apparatus of claim 13, wherein the light emitting module further comprises a light emitting panel having a length greater than a width, and
a first light source that emits one type of ray and a second light source that emits another type of ray among the plurality of light emitting elements are alternately arranged in a longitudinal direction of the light emitting panel.

16. The virus inactivation apparatus of claim 13, wherein the light emitting module further comprises a light emitting panel having a cylindrical shape and
the plurality of light emitting elements are arranged on an inner circumferential surface of the light emitting panel at preset intervals.

17. The virus inactivation apparatus of claim 13, wherein the plurality of light emitting elements comprises at least one type of light source between the UV-C light source and the visible light source and
the virus inactivation kit comprises at least one chemical selected from methylene blue, thiazole orange, thioperielium, and dipyramol.

18. The virus inactivation apparatus of claim 13, wherein the plurality of light emitting elements comprises at least one type of light source between the UV-A light source and the UV-C light source and
the virus inactivation kit comprises at least one chemical selected from 4-aminomethylenetrioxalene and riboflavin.
